# EUROPEAN PATENT APPLICATION

(11) **EP 4 759 148 A1**
(43) Date of publication of application: **17.06.2026**
(21) Application number: 24851855.7
(22) Date of filing: 06.08.2024
(51) Int. Cl.: A23L 33/10, A23L 33/135, A61K 31/194, A61K 35/744, A61P 43/00

(54) **ADJUSTMENT OF CIRCADIAN RHYTHM**

(30) Priority: 08.08.2023 JP 2023129595
(71) Applicant: MEIJI CO., LTD, Chuo-ku Tokyo 104-8306 (JP)
(72) Inventor: YAMADA, Naruomi, Hachioji-shi, Tokyo 192-0919 (JP); MIZOGUCHI, Chinami, Hachioji-shi, Tokyo 192-0919 (JP)
(74) Representative: TBK
(86) International application number: PCT/JP2024/028102
(87) International publication number: WO 2025/033432

(57) **Abstract**

The present disclosure provides compounds, compositions, and methods effective in regulating the circadian rhythm. More specifically, the present disclosure provides compounds, compositions, and methods useful for ameliorating the circadian rhythm disruption of delayed sleep-phase type. The composition of the present disclosure contains as an active ingredient at least one selected from the group consisting of fumaric acid and salts thereof.

## Description

### Technical Field

The present disclosure relates to the regulation of circadian rhythms. More specifically, the present disclosure relates to a compound effective in ameliorating delayed-sleep-phase-type circadian rhythm disruption, and a composition containing the compound.

### Background Art

Living organisms possess a function that actively changes their internal environment in a cycle of about 24 hours (one day), synchronizing with the 24-hour day-night cycle caused by the Earth's rotation. It is known that humans also exhibit rhythms with a cycle of about 24 hours in basic bodily functions, such as body temperature and hormone secretion, and this periodic rhythm is called "circadian rhythm." Circadian rhythms are also observed even when organisms remain at rest with no changes in light or temperature, indicating that organisms possess a timekeeping mechanism within their bodies referred to as the "circadian clock". The circadian clock in mammals, including humans, is known to be located in the suprachiasmatic nucleus of the hypothalamus, situated at the lower central part of the brain. Recent molecular biology research has revealed that in the circadian clock cells, multiple genes, known as clock genes, synthesize clock proteins, which bind to each other and are degraded in a repeatedly occurring cycle of about 24 hours, and that such genetic activities determine the circadian rhythm of the circadian clock. Moreover, in addition to the sleep-wake rhythm, various biorhythms are known as examples of biological phenomena that mark the circadian rhythm, such as appetite, sexual desire, blood pressure and body temperature rhythms, endocrine system rhythms (e.g., secretion of melatonin, adrenocortical hormones, and growth hormones), metabolic rhythms, immune system rhythms, and autonomic nervous system rhythms.

The period of the human circadian clock is slightly longer than 24 hours (although a small number of people have shorter periods), so the human circadian rhythm is set to synchronize the timing of the internal clock with the external 24-hour light-dark cycle (this is called the "entrainment mechanism"). Due to this entrainment mechanism functioning, humans are able to align their circadian clock with changes in the light-dark cycle caused by seasonal variations in day length due to Earth's revolution or rapid travel to different time zones. For example, traveling long distances in a short time by airplane for overseas business or overseas leisure trips often results in a phase shift (time difference) between daytime and nighttime at the departure and arrival locations, frequently causing what is known as "jet lag." Jet lag disrupts the sleep-wake rhythm until the circadian rhythm gradually adjusts to the time zone of the destination, leading to temporary declines in physical condition, such as decreased attention and concentration, as well as various disturbances in the biorhythms as mentioned above.

Recently, a condition similar to such jet lag, termed "social jetlag" has become a matter of concern. Social jet lag refers to a jet-lag phenomenon that occurs in everyday life due to variations in daily rhythms caused by shift work or other factors, as well as differences in wake-up and bedtime hours between weekdays and weekends or differences in sleep duration. Social jet lag, which is caused in part by circadian rhythm disruption, has been reported to not only induce declines in physical condition, such as daytime sleepiness, decreased cognitive functions, and fatigue, but also lead to various mental and physical health issues, including exacerbation of metabolic syndrome symptoms, increased depression, and sleep disorders.

Circadian rhythm sleep-wake disorder, one of the sleep disorders, is mainly classified into three types. The first is the "delayed sleep-phase type" with which one cannot sleep from late night until dawn and cannot wake up until around noon or evening the next day. The second is the "advanced sleep-phase type" with which one becomes sleepy from evening or early night and wakes up early in the morning. The third is the "non-24-hour sleep-wake type" with which the bedtime and the waking time shift every day. Among these, the delayed sleep-phase type is the most common. Currently, light therapy is a standard method as a treatment method to ameliorate these symptoms. Additionally, the administration of melatonin, which is an endogenous hormone that regulates the circadian rhythm, or its precursors (e.g., serotonin and tryptophan) has also been studied. Other studies also report that ornithine (PTL 1), substances that inhibit vasopressin receptors V1a and V1b (PTL 2), cinobufagin compounds contained in the crude drug *Senso* (dried secretions collected from the parotid or sebaceous glands of toads etc.) (PTL 3), cinnamic acid compounds contained in the crude drug cinnamon bark (PTL 4), and (1R,2S)-2-(((2,4-dimethylpyrimidin-5-yl)oxy)methyl)-2-(3-fluorophenyl)-N-(5-fluoropyridin-2-yl)cyclopropanecarboxamide and its pharmaceutically acceptable salts (PTL 5) have the action of regulating the circadian rhythm, and are expected to be effective in ameliorating the circadian rhythm sleep-wake disorder.

### Citation List

### Patent Literature

PTL 1: JP2019-182775A
PTL 2: WO2019/176220A
PTL 3: JP2016-204280A
PTL 4: JP2016-204281A
PTL 5: JP2021-120410A

### Non-patent Literature

NPL 1: Molecular components of the mammalian circadian clock, Buhr E. D., Takahashi J. S. Handb Exp Pharmacol. 2013; (217):3-27
NPL 2: Tahara Y. et al., Gut Microbiota-Derived Short Chain Fatty Acids Induce Circadian Clock Entrainment in Mouse Peripheral Tissue. Sci Rep. 2018; 8(1) :1395.
NPL 3: Yamajuku D. et al., Real-time monitoring in three-dimensional hepatocytes reveals that insulin acts as a synchronizer for liver clock. Sci Rep. 2012; 2:439. doi: 10.1038/srep00439
NPL 4: E. Yamamoto et al., Effect of fumaric acid on the growth of Lactobacillus delbrueckii ssp. bulgaricus during yogurt fermentation, J Dairy Sci. 2021 Sep; 104(9): 9617-9626.
NPL 5: V. Gokmen & J. Acar, Fumaric acid in apple juice: a potential indicator of microbial spoilage of apples used as raw material: Food Additives & Contaminants: Vol. 21, No. 7, 2004, pp.626-631 (tandfonline.com)

### Summary of Invention

### Technical Problem

An object of the present disclosure is to provide compounds, compositions, and methods used for regulating the circadian rhythm. Additionally, another object of the present disclosure is to provide compounds, compositions, and methods effectively used for ameliorating the circadian rhythm disruption of delayed sleep-phase type.

### Solution to Problem

In recent molecular biological studies of the circadian clock, especially on clock genes, in vitro test methods have been frequently used to investigate action on the circadian clock (circadian regulatory action) by monitoring the circadian rhythm of luciferase activity using mammalian cells transfected with reporter plasmids in which luciferase genes are linked to the promoters of clock genes, such as Period 1 (Perl), Period 2 (Per2), and Bmal1, as an alternative to animal experiments. The present inventors conducted extensive research using established in vitro test methods in order to achieve the objects and found that fumaric acid, which is a low-molecular-weight organic acid, has the action of regulating the circadian rhythm, in particular, the action of advancing the phase of the circadian rhythm and the action of shortening the cycle of the circadian rhythm.

The present inventors also found that fumaric acid has the action of selectively enhancing the gene expression of short-chain fatty acid receptor GPR41 in enteroendocrine cells, which is known to be involved in the secretion of the appetite-suppressing hormone peptide YY (PYY) and the insulin secretion-promoting hormone GLP-1. This suggests that fumaric acid not only regulates the circadian rhythm by acting on clock genes in the central nervous system, but also potentially regulates the circadian rhythm of peripheral tissues through insulin by promoting GLP-1 secretion via GPR41 in intestinal cells.

The present disclosure has been completed through further investigation based on these findings, and encompasses the following embodiments.
(I) A composition for regulating the circadian rhythm
   (I-1) A composition for regulating a circadian rhythm, comprising as an active ingredient at least one selected from the group consisting of fumaric acid and salts thereof.
   (I-2) The composition for regulating a circadian rhythm according to (I-1), which is for use in advancing the phase of the circadian rhythm and/or in shortening the cycle of the circadian rhythm.
   (I-3) The composition for regulating a circadian rhythm according to (I-1) or (I-2), comprising a fermented material of a fumaric acid-producing bacterium or a processed product thereof, the fermented material or the processed product thereof containing at least one selected from the group consisting of fumaric acid and salts thereof.
   (I-4) The composition for regulating a circadian rhythm according to (I-3), wherein the fumaric acid-producing bacterium is at least one species of lactic acid bacteria selected from the group consisting of *Streptococcus thermophilus* and *Lactiplantibacillus plantarum*.
   (I-5) The composition for regulating a circadian rhythm according to any one of (I-1) to (I-4), which is a composition for regulating a daily rhythm.
   (I-6) The composition for regulating a circadian rhythm according to any one of (I-1) to (I-5), which is a food or drink, a quasi-drug, a drug, or an additive therefor.
   (I-7) The composition for regulating a circadian rhythm according to any one of (I-1) to (I-6), comprising neither the following compound (1) nor the following compound (2):
      (1) a fumarate salt of (1R,2S)-2-(((2,4-dimethylpyrimidin-5-yl)oxy)methyl)-2-(3-fluorophenyl)-N-(5-fluoropyridin-2-yl)cyclopropanecarboxamide, and
      (2) a fumarate salt of ornithine.
(II) Use for the production of a composition for regulating the circadian rhythm
   (II-1) Use of at least one compound selected from the group consisting of fumaric acid and salts thereof, or of a composition containing the at least one compound, for the production of a composition for regulating a circadian rhythm.
   (II-2) The use according to (II-1), wherein the composition containing the at least one compound selected from the group consisting of fumaric acid and salts thereof is a fermented material of a fumaric acid-producing bacterium or a processed product thereof.
   (II-3) The use according to (II-2), wherein the fumaric acid-producing bacterium is at least one species of lactic acid bacteria selected from the group consisting of *Streptococcus thermophilus* and *Lactiplantibacillus plantarum*.
   (II-4) The use according to any one of (II-1) to (II-3), wherein the composition for regulating a circadian rhythm is a quasi-drug, a drug, a non-therapeutic food or drink for a human or non-human mammal in a healthy or pre-disease state, or an additive therefor.
   (II-5) The use according to (II-4), wherein the human in a healthy or pre-disease state is a human having at least one symptom selected from the group consisting of the following 1) to 4) :
      1) a risk of developing a circadian rhythm disorder,
      2) a circadian rhythm sleep disorder,
      3) a desynchronized circadian rhythm, and
      4) a poor physical condition due to circadian rhythm disruption. (II-6) The use according to any one of (II-1) to (II-5), wherein the at least one compound is neither the following compound (1) nor the following compound (2), and wherein the composition comprises neither the following compound (1) nor the following compound (2):
         (1) a fumarate salt of (1R,2S)-2-(((2,4-dimethylpyrimidin-5-yl)oxy)methyl)-2-(3-fluorophenyl)-N-(5-fluoropyridin-2-yl)cyclopropanecarboxamide, and
         (2) a fumarate salt of ornithine.
(III) A compound for use in the regulation of the circadian rhythm and a composition containing the compound
   (III-1) At least one compound selected from the group consisting of fumaric acid and salts thereof, or a composition containing the at least one compound, for use in the regulation of a circadian rhythm.
   (III-2) The at least one compound or composition containing the at least one compound for use according to (III-1), wherein the composition is a fermented material of a fumaric acid-producing bacterium or a processed product thereof.
   (III-3) The at least one compound or composition containing the at least one compound for use according to (III-2), wherein the fumaric acid-producing bacterium is at least one species of lactic acid bacteria selected from the group consisting of *Streptococcus thermophilus* and *Lactiplantibacillus plantarum.*
   (III-4) The at least one compound or composition containing the at least one compound for use according to any one of (III-1) to (III-3), wherein the at least one compound is neither the following compound (1) nor the following compound (2), and wherein the composition comprises neither the following compound (1) nor the following compound (2):
      (1) a fumarate salt of (1R,2S)-2-(((2,4-dimethylpyrimidin-5-yl)oxy)methyl)-2-(3-fluorophenyl)-N-(5-fluoropyridin-2-yl)cyclopropanecarboxamide, and
      (2) a fumarate salt of ornithine.
(IV) A method for regulating the circadian rhythm
   (IV-1) A method for regulating a circadian rhythm of a mammal,
      comprising administering to the mammal
      at least one compound selected from the group consisting of fumaric acid and salts thereof, or
      a composition containing the at least one compound.
   (IV-2) The method for regulating a circadian rhythm according to (IV-1), wherein the composition is a fermented material of a fumaric acid-producing bacterium or a processed product thereof.
   (IV-3) The method for regulating a circadian rhythm according to (IV-2), wherein the fumaric acid-producing bacterium is at least one species of lactic acid bacteria selected from the group consisting of *Streptococcus thermophilus* and *Lactiplantibacillus plantarum.*
   (IV-4) The method for regulating a circadian rhythm according to any one of (IV-1) to (IV-3), wherein the mammal is a human or non-human animal in a healthy or pre-disease state, and the method is a non-therapeutic method for the mammal.
   (IV-5) The method for regulating a circadian rhythm according to any one of (IV-1) to (IV-4), wherein the mammal is a human having at least one symptom selected from the group consisting of the following 1) to 4):
      1) a risk of developing a circadian rhythm disorder,
      2) a circadian rhythm sleep disorder,
      3) a desynchronized circadian rhythm, and
      4) a poor physical condition due to circadian rhythm disruption. (IV-6) The method for regulating a circadian rhythm according to any one of (IV-1) to (IV-5), wherein the at least one compound is neither the following compound (1) nor the following compound (2), and wherein the composition comprises neither the following compound (1) nor the following compound (2):
         (1) a fumarate salt of (1R,2S)-2-(((2,4-dimethylpyrimidin-5-yl)oxy)methyl)-2-(3-fluorophenyl)-N-(5-fluoropyridin-2-yl)cyclopropanecarboxamide, and
         (2) a fumarate salt of ornithine.

### Advantageous Effects of Invention

The present disclosure enables the regulation of circadian rhythms. More preferably, the compound of the present disclosure, a composition containing the compound, or methods using the compound or composition exert the action of advancing the phase of the circadian rhythm and/or the action of shortening the cycle of the circadian rhythm on the circadian rhythm disruption of delayed sleep-phase type, and thus ameliorate the circadian rhythm disruption. Therefore, the present disclosure is expected to ameliorate circadian rhythm sleep disorders, such as delayed sleep phase syndrome caused by circadian rhythm disruption, disturbances in daily rhythm caused by circadian rhythm disruption, and declines in physical condition arising from disturbances in circadian rhythms and daily rhythms.

### Brief Description of Drawings

Fig. 1 shows the results of evaluating the changes in circadian rhythm by measuring the luminescence intensity of luciferase over time using per2 reporter cells with fumaric acid added (Fumaric Acid) or without fumaric acid (Control) in Experimental Example 1.
Fig. 2 shows the results of evaluating the effects of fumaric acid on the expression of GPR41 gene in Caco-2 cells (human colon cancer-derived cells) in Experimental Example 2.

### Description of Embodiments

### (I) A composition for regulating the circadian rhythm

The composition for regulating the circadian rhythm of the present disclosure (simply "the composition" below) contains, as an active ingredient, at least one compound selected from the group consisting of fumaric acid and salts thereof. Fumaric acid and salts thereof are collectively referred to as "Fumarates" below.

The salts of fumaric acid (fumarate salts) can be any pharmaceutically acceptable or edible salt that brings about the effects of the present disclosure; the fumarate salt include metal salts of fumaric acid (e.g., complex salts of alkali metals, such as sodium, potassium, and lithium, with fumaric acid); alkaline earth metal salts of fumaric acid (e.g., complex salts of alkaline earth metals, such as magnesium or calcium, with fumaric acid); hydrogen metal salts of fumaric acid (e.g., complex salts of metal hydrogen, such as sodium hydrogen or potassium hydrogen, with fumaric acid); and complex salts of fumaric acid with ammonium salts (e.g., ammonium salts and tetramethylammonium salts).

However, the fumarate salt of the present disclosure excludes the following compounds:
(1) a fumarate salt of (1R,2S)-2-(((2,4-dimethylpyrimidin-5-yl)oxy)methyl)-2-(3-fluorophenyl)-N-(5-fluoropyridin-2-yl)cyclopropanecarboxamide, and
(2) a fumarate salt of ornithine.

Compound (1) is a fumarate salt of Lemborexant (generic name) represented by the following formula (I).

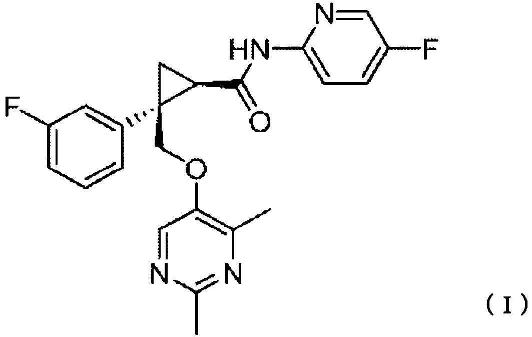

The composition is used to regulate the circadian rhythm of mammals, including humans.

The "circadian rhythm" includes the expression rhythm of genes, including clock genes. Thus, the composition also encompasses compositions used to regulate the expression rhythm of genes, including clock genes.

Clock genes are genes that govern the biorhythm, particularly circadian rhythm, and are also referred to as "circadian regulatory genes." Clock genes known to exist in mammals, including humans, include Period (per genes: e.g., per1 and per2), Cryptochrome (cry genes: e.g., cry1 and cry2), Clock, Dec, Casein Kinase 1 (CK1 genes: CK1ε and CK1δ), and Bmal (Bmal genes: e.g., Bmal1).

The clock gene targeted by the present disclosure is preferably "per2 gene."

The circadian rhythm is established by a negative feedback loop mechanism between the clock genes, with the per2 gene playing a central role (NPL 1: Molecular components of the mammalian circadian clock, Buhr E. D., Takahashi J. S. Handb Exp Pharmacol. 2013; (217): 3-27). Thus, controlling the expression rhythm of the per2 gene leads to controlling the expression of a series of clock genes, resulting in the regulation of the circadian rhythm as a biorhythm. Thus, the composition may be used to regulate the expression rhythm of a series of clock genes through the regulation of the expression rhythm of the per2 gene. Additionally, the composition can be used to regulate the circadian rhythm as a biorhythm by regulating the expression rhythm of a series of clock genes through the regulation of the expression rhythm of the per2 gene.

In the present disclosure, "regulating the circadian rhythm" includes the meaning of "regulating the phase of the circadian rhythm" and/or "regulating the cycle of the circadian rhythm."

The phrase "regulating the phase of the circadian rhythm" includes the meaning of "regulating the light-dark phase of the circadian rhythm by regulating the phase of the expression rhythm of a clock gene." The clock gene referred to here is preferably the per2 gene.

The phrase "regulating the phase of the expression rhythm of a clock gene" means regulating the temporal change in the phase of the periodic expression rhythm of the gene, which is controlled by the activation and/or suppression of clock gene expression at regular intervals. The Fumarates, which are the active ingredients of the composition, have the action of regulating the phase of the expression rhythm of the clock gene (per2 gene) by advancing the phase of the expression rhythm of the clock gene chronologically (in other words, along the time axis), as shown in Experimental Example 1, described below. This is also referred to as "phase-advancing action." Through the phase-advancing action of Fumarates, the composition enables the advancement (shifting forward) of the light-dark phase of the circadian rhythm along the time axis by advancing (shifting forward) the phase of the expression rhythm of the clock gene (per2 gene) along the time axis, thereby allowing for the regulation of the circadian rhythm.

The phrase "regulating the cycle of the circadian rhythm" includes regulating the light-dark cycle of the circadian rhythm by regulating the length of one cycle of the expression rhythm of a clock gene. The clock gene referred to here is preferably the per2 gene. The term "cycle" refers to the time or duration (a time period to be repeated) it takes for a cyclical phenomenon or action to complete one cycle and return to the same state as before.

The phrase "regulating the length of one cycle of the expression rhythm of a clock gene" means regulating the length (length of one cycle: period) of the periodic expression rhythm of a gene, which is controlled by the activation and/or suppression of clock gene expression at regular intervals. Fumarates, which are the active ingredients of the composition, have the action of regulating the cycle of the expression rhythm of the clock gene (per2 gene) by shortening the length per cycle of the expression rhythm, as shown in Experimental Example 1 below. This is also referred to as "cycle-shortening action." Through the cycle-shortening action of Fumarates, the composition can shorten the light-dark cycle of the circadian rhythm by shortening the cycle of the expression rhythm of a clock gene (per2 gene), thereby allowing for the regulation of the circadian rhythm.

Furthermore, as shown in Experimental Example 2 described below, Fumarates, which are the active ingredient of the composition, exert the action of inducing gene expression of GPR41, one of the short-chain fatty acid receptors present in intestinal cells. GPR41 is known to be involved in the secretion of energy metabolism-related intestinal hormones, such as the insulin secretion-promoting hormone GLP-1 (NPL 2). Thus, Fumarates are thought to exert their action of promoting the secretion of GLP-1 and insulin in peripheral tissue by inducing the expression of the GPR41 gene. It is known that dietary hormones, such as insulin, function as synchronizing factors for the circadian rhythm in peripheral tissue (NPL 3). Therefore, the composition containing Fumarates enables the regulation of the circadian rhythm by promoting insulin secretion through the induction of GPR41 gene expression in peripheral tissue.

Thus, the "regulation of the circadian rhythm" as referred to in the present disclosure includes regulation in the central tissues and/or regulation in the peripheral tissue. The composition can regulate the circadian rhythm through regulation in both central and peripheral tissues through the action of Fumarates shown in Experimental Examples 1 and 2. In the present disclosure, the term "regulation" also includes the meanings of "adjustment" and "control."

The composition can regulate daily rhythms through the regulation of the circadian rhythm. Thus, the composition can also be used as a composition for regulating the daily rhythm. The phrase "daily rhythm" as used here refers to the rhythm of daily life based on an individual's unique circadian rhythm, that is, the way of spending time (behavioral rhythm). Specifically, the daily rhythm refers to a rhythm in which various elements of life, such as sleep, wakefulness, activity, rest, and eating, are repeated in a certain cycle based on the circadian rhythm, while being influenced by the environment.

The composition encompasses food or drink, quasi-drugs, and drugs used for regulating the circadian rhythm or daily rhythm. The food or drink includes non-therapeutic food and drink for humans. The food or drink also includes not only those for humans, but also food products for non-human animals, such as pets and livestock (including feed, fodder, and experimental feed). Additionally, drugs and quasi-drugs include not only those for humans, but also those for non-human animals.

The subjects to which the food or drink, quasi-drugs, or drugs used for regulating the circadian rhythm or daily rhythm are applied are mammals including humans, and preferably humans. In the present disclosure, humans include those in a healthy state and those in a pre-disease state who have physical or mental discomfort but have not yet developed a definitive illness.

The composition can be suitably applied to, but not limited to, for example, humans with at least one of the following symptoms 1) to 4):
1) a risk of developing a circadian rhythm disorder: for example, shift workers, those who work long hours, overseas travelers, or individuals with irregular living hours due to staying up late or other reasons,
2) a circadian rhythm sleep disorder, such as delayed sleep phase syndrome,
3) a desynchronized circadian rhythm due to conditions such as blindness, and
4) a poor physical condition due to circadian rhythm disruption, in particular a delay in the phase of the circadian rhythm and/or an elongation of the cycle of the circadian rhythm.

The poor physical condition described in 4) includes physical ailments, such as drowsiness, headache, head heaviness, loss of appetite, easy fatigability, lethargy, disruption of metabolic rhythm, endocrine disorders, and/or gastrointestinal dysfunction.

The poor physical condition also includes mental ailments, such as decreased concentration or motivation, melancholy, anxiety, and/or feelings of depression. The humans include individuals in a healthy state and those in a pre-disease state.

The composition includes additives for preparing food or drink, quasi-drugs, or drugs used to regulate the circadian rhythm or daily rhythm. Food or drink, quasi-drugs, or drugs can be prepared by using the composition for regulating the circadian rhythm as an additive to impart a circadian rhythm modulating effect to the food or drink, quasi-drugs, or drugs. Accordingly, food or drink, quasi-drugs, or drugs with a circadian rhythm modulating effect can be prepared for regulating the circadian rhythm.

The origin of Fumarates used as the active ingredient of the composition is not particularly limited, and the Fumarates may be chemically synthesized products or those extracted and isolated from natural sources (animals, plants, microorganisms, and substances produced by these). Fumarates may also be those extracted and isolated from fermented materials prepared by using microorganisms (fumaric acid-producing bacteria). Further, Fumarates to be contained in the composition may be of any purity, including crude purified products, as long as they do not impede the effects of the present invention. For example, as long as the effects of the present disclosure are not impaired, a fermented material containing Fumarates prepared using microorganisms or a processed product thereof can be used as is, as an active ingredient of the composition for regulating the circadian rhythm.

Examples of microorganisms as used here include bacteria having the ability to produce Fumarates. In the present disclosure, bacteria having the ability to produce Fumarates are collectively referred to as "fumaric acid-producing bacteria." Examples of fumaric acid-producing bacteria include, but are not limited to, lactic acid bacteria as long as they have the ability to produce Fumarates. Such lactic acid bacteria include, but are not limited to, preferably lactic acid bacteria belonging to the genus *Streptococcus* and lactic acid bacteria belonging to the genus *Lactiplantibacillus.* Lactic acid bacteria belonging to the genus *Streptococcus* include *S. thermophilus,* and lactic acid bacteria belonging to the genus *Lactiplantibacillus* include *L. plantarum* (see, for example, NPL 4 and 5). The fermented material prepared by culture using these lactic acid bacteria contains Fumarates. Thus, the fermented material or a processed product thereof (collectively referred to as "Fumarates -containing fermented product") can be used as an active ingredient of the composition, or can be used as is, as the composition. The processed product refers to a product made by subjecting a fermented material prepared using fumaric acid-producing bacteria to processing; for example, the processed product includes, without limitation, processed products prepared through various treatments (e.g., concentration, purification, and drying) so as to contain Fumarates at high concentrations or high purity.

The content of Fumarates in the composition can be appropriately selected from a range of, for example, 0.1 to 99 mass%, to the extent that the composition exhibits a circadian regulatory action.

Whether the composition has the circadian regulatory action can be confirmed according to the method in Experimental Example 1, described below. Specifically, it can be evaluated by examining the effect of a candidate composition on the expression cycle of a reporter gene (e.g., luciferase gene) using cultured cells (e.g., human osteosarcoma-derived cells) into which the reporter gene under expression control by a clock gene promoter (e.g., per2 promoter) has been introduced. In this test, when the expression phase of the reporter gene advances and/or the expression cycle shortens in the presence of the candidate composition as compared to the case in the absence of the candidate composition (control), the candidate composition can be determined to have circadian regulatory action.

Instead of or in addition to this evaluation method, the method in Experimental Example 2, described below, can also be used for evaluation. Specifically, cultured cells of peripheral tissue that can express the GPR41 gene (e.g., human colon cancer-derived cells) are used to examine the effect of a candidate composition on the expression of the gene for evaluation. In this test, when the expression of the GPR41 gene increases in the presence of the candidate composition as compared to the case in the absence of the candidate composition (control), the candidate composition is determined to have circadian regulatory action.

### (A) Application to food or drink, or additives for food or drink

In one embodiment of the present disclosure, the composition includes food or drink, or additives for food or drink. The form of the food or drink, or of the additives for food or drink is not particularly limited, and may be liquid, semi-liquid (including paste, gel, and sol forms), or solid, and also encompasses forms such as beverages. Additionally, the food or drink includes health foods (including functional foods, supplemental foods, supplements, food with health claims bearing disease risk reduction claims (foods for specified health uses, foods with nutrient function claims, foods with function claims), and foods for patients), as well as other general food and drink items. Health foods refer to food or drink consumed with the expectation of maintaining or improving health. Supplements refer to food or drink in a formulation form containing an enriched specific ingredient (Fumarates). These foods and drinks are non-therapeutic foods and drinks that are not intended for treatment purposes.

For example, the food or drink may be a dairy product, such as yogurt and cheese produced by subjecting a food material like milk to microbial fermentation to form Fumarates, or may be a koji-fermented food product. For supplements, the composition can be made into various forms, such as tablets produced by adding excipients, binders, etc. to the composition, followed by kneading and then tableting, powders (powdered medicines), pills, capsules, jellies, or granules. In addition to Fumarates, the composition may appropriately contain food-grade (edible) carbohydrates, proteins, lipids, vitamins, minerals, sugars (e.g., glucose), natural or artificial sweeteners, citric acid, carbonated water, fruit juice, stabilizers, preservatives, binders, thickeners, and/or emulsifiers.

For the food or drink in a beverage form, examples of non-alcoholic beverages include, but are not limited to, mineral water, near-water, sports drinks, tea beverages, milk beverages, coffee beverages, fruit juice beverages, vegetable juice beverages, fruit and vegetable juice beverages, and carbonated beverages. The food or drink in a beverage form may be a beer beverage with an alcohol content of less than 1%, such as a non-alcoholic beer.

The tea beverages among the non-alcoholic beverages refer to drinks made by decocting leaves (tea leaves) from tea plants, which are evergreen trees of the *Theaceae* family, or leaves from plants other than tea plants, or grains, etc., for drinking purposes. The tea beverages include fermented teas, semi-fermented teas, and non-fermented teas. Specific examples of tea beverages include Japanese tea (e.g., green tea and barley tea), black tea, herbal tea (e.g., jasmine tea), Chinese tea (e.g., Chinese green tea and oolong tea), and *hojicha* (roasted green tea). The milk beverages refer to drinks that are primarily made from raw milk, cow milk, other milk, or food products produced by using these kinds of milk as ingredients. The milk beverages include not only those made from cow milk or similar materials, but also those made from processed milk, such as nutrient-fortified milk, flavored milk, and sweetened hydrolyzed milk.

When the composition is in the form of food or drink, the intake per adult per day can be adjusted within a range of 6 mg/kg or less in terms of fumaric acid intake. For example, for an adult weighing 60 kg, the daily intake can be within a range of 20 to 360 mg. Similarly, when the composition is formulated into individual containers as a daily serving, the amount of the composition in a container can be, for example, within a range of 20 to 360 mg in terms of daily intake of fumaric acid for an adult weighing 60 kg.

Moreover, the composition in a form of food or drink can be produced according to a standard production method for various foods or drinks, except for including the step of adding and blending Fumarates or a composition containing Fumarates as one of the ingredients (e.g., as an additive for food and drink).

### (B) Application to quasi-drugs, drugs, or additives therefor

In one embodiment of the present disclosure, the composition includes quasi-drugs, drugs (which are collectively referred to as "the drug etc." below), or additives therefor. These can be produced, for example, by mixing Fumarates as the active ingredient with pharmaceutically acceptable carriers or additives, such as carriers, excipients, binders, and diluents, according to a standard method. These may be in the form of orally administered preparations or parenterally administered preparations. Oral dosage forms include granules, powders, tablets, pills, capsules, and syrup. Parenteral dosage forms include injections, infusions, nasally administered preparations, and enteric (tube feeding) preparations. However, the dosage form is not limited to these forms. Preferably, the dosage form is an oral dosage form.

The pharmaceutically acceptable carriers include excipients and diluents. In addition to the carriers, additives, such as preservatives, stabilizers, binders, pH adjusting agents, buffers, thickeners, gelling agents, and antioxidants, can optionally be added. These additives are preferably those used in pharmaceutical manufacturing.

The drug etc. of the present disclosure may be used in combination with other ingredients that have circadian rhythm modulating effects, in particular, the action of advancing the phase of the circadian rhythm, and/or the effect of shortening the cycle of the circadian rhythm.

The dosage of the drug etc. of the present disclosure may vary depending on the form of administration, the patient's age, weight, nature or severity of the symptoms to be treated, etc.; a preferable dosage for oral administration per adult per day can be within a range of 20 to 360 mg in terms of the dosage of fumaric acid, for example, for an adult weighing 60 kg. The dosage may be appropriately selected according to various conditions, such as the route of administration. Administration to a patient can be performed once daily or multiple times daily.

The target patients are typically humans, but may also include non-human mammals, such as pet animals like dogs. The non-human animals are preferably mammals that are diurnal, like humans.

### (II) Use for the production of a composition for regulating the circadian rhythm

The present disclosure encompasses the use of Fumarates and the use of a composition containing Fumarates in the production of a composition for regulating the circadian rhythm.

As mentioned above, "the composition for regulating the circadian rhythm" refers to a composition used to regulate the circadian rhythm or daily rhythm of mammals, including humans, and encompasses non-therapeutic food or drink, quasi-drugs, and drugs. The composition for regulating the circadian rhythm also encompasses additives for preparing the non-therapeutic food or drink, quasi-drugs, or drugs. The phrases "Fumarates" and "composition containing Fumarates" are as described above, and the description in section (I) above can be incorporated by reference.

### (III) The compound for use in the regulation of the circadian rhythm and the composition containing the compound

The present disclosure encompasses Fumarates and a composition containing Fumarates for use in the regulation of the circadian rhythm. The phrases "the regulation of the circadian rhythm" "Fumarates" and "composition containing Fumarates" are as described above, and the description in section (I) above can be incorporated by reference. As stated in section (I), the regulation of the circadian rhythm includes the regulation of the daily rhythm.

### (IV) The method for regulating the circadian rhythm

The present disclosure encompasses a method for regulating the circadian rhythm of a mammal by administering Fumarates or a composition containing Fumarates to the mammal. The terms "mammal" "Fumarates" "composition containing Fumarates" "administering" and "regulating the circadian rhythm" are as described above, and the description in section (I) above can be incorporated by reference. As stated in section (I), the regulation of the circadian rhythm includes the regulation of the daily rhythm.

In the present specification, the terms "comprise" "include" and "contain" encompass the meanings of consisting of and consisting essentially of.

### Examples

To aid in understanding the elements and effects of the present disclosure, the present invention is explained with reference to Experimental Examples. However, the present disclosure is not limited in any way by these Experimental Examples. The following experiments were conducted at room temperature (25±5°C) under atmospheric pressure unless otherwise specified. Unless otherwise specified, the units "%" and "parts" below refer to mass% and parts by mass, respectively.

The materials used in the following Experimental Examples and their sources are as follows:
- Fumaric acid: fumaric acid, Wako reagent (Fujifilm Wako Pure Chemical Corporation: Product Code 069-00652)
- NanoLuc (registered trademark) luciferase vector: a vector for expressing luciferase derived from *Oplophorus gracilirostris.* pNL2.2 NlucP Hygro Vector, which has hygromycin resistance and a PEST sequence (Promega Corporation).
- Transfection reagent 1: ViaFect (trademark) Transfection Reagent (Promega Corporation)
- U-2OS cells: an osteosarcoma-derived cell line obtained from a 15-year-old Caucasian female (ATCC (Cat. No. HTB-96)).
- Endurazine: a substrate for luciferase reporter assays using a NanoLuc (registered trademark) luciferase vector (Promega Corporation)
- High-glucose medium 1: D-MEM (high glucose) (containing L-glutamine and phenol red) (manufactured by Fujifilm Wako Pure Chemical Corporation: product code 044-29765)
- High-glucose medium 2: D-MEM (high glucose) (containing L-glutamine and HEPES) (manufactured by Fujifilm Wako Pure Chemical Corporation: product code 044-32955)
- 1% Penicillin-streptomycin solution (GIBCO 15140): manufactured by Thermo Fisher Scientific
- Long-term real-time luminescence measurement system for cultured cells: WSL-1565, Kronos HT (measurement method: weak light measurement on a photon counting mode using photomultiplier tubes) (manufactured by Ikeda Scientific Co., Ltd.)
- Caco-2 cells: human colon cancer-derived epithelial cell line (Abcam)

### Experimental Example 1: Evaluation of the circadian rhythm using per2 reporter cells

Referring to the "Cell-based circadian assays" described in the article by H. Oike et al. (NPL 1), a circadian rhythm assay (luciferase reporter assay) using per2 reporter cells was performed to evaluate the action of fumaric acid on circadian rhythms.

### (1) Preparation of per2 reporter cells

U-2OS cells were transfected with a vector prepared by incorporating the per2 promoter region into a NanoLuc (registered trademark) luciferase vector by using transfection reagent 1 according to a standard method. After about 24 hours, the medium was replaced, and dexamethasone was added at a final concentration of 100 nM to synchronize the cells. After two hours, 1% substrate (Endurazine) was added, and the luminescence intensity of luciferase was measured every 30 minutes using a plate reader equipped with CO₂ control and temperature control.

The cells in the wells that showed a regular rhythm in luminescence intensity in the measurement were first proliferated in a 24-well plate, then seeded (5 × 10⁴ cells/10 cm dish), and subjected to hygromycin selection to form hygromycin-resistant colonies. After 25 days, formed colonies were collected and seeded in a 96-well plate (1 × 10³ cells/well). After three days, the luminescence intensity of luciferase was measured every 60 minutes using a plate reader with CO² control and temperature control, in the same manner as described above.

Cell lines that exhibited circadian rhythms with stable luminescence intensity in this measurement were further subjected to limiting dilution and cloning. Subsequently, cell lines with minimal variation in circadian rhythm cycle and stable periodicity were selected. These selected cell lines, referred to as "per2 reporter cells" were used in the following experiments.

### (2) Evaluation of the circadian rhythm with fumaric acid using per2 reporter cells

The prepared per2 reporter cells were seeded in a 96-well plate at a density of 5 × 10³ cells/well. The medium used was high-glucose medium 1 supplemented with 10% fetal bovine serum (FBS), 1% penicillin-streptomycin, and hygromycin, and the cells were cultured at 37°C under 5% CO₂ conditions.

After two days from culture, 100 nM dexamethasone was added, and the cells were treated for 2 hours, followed by adding a 1% substrate (Endurazine). Subsequently, the luminescence intensity of luciferase was measured every 12 minutes. For the measurement, high-glucose medium 2 supplemented with 5% FBS and 1% penicillin-streptomycin was used.

Subsequently, fumaric acid was added to the measurement medium to achieve a final concentration of 5 mM, and the luminescence intensity of luciferase was measured over time using a long-term real-time luminescence measurement system for cultured cells (WSL-1565, Kronos HT) to evaluate changes in circadian rhythm. For a control, the luminescence intensity of luciferase in per2 reporter cells cultured in measurement medium without fumaric acid was measured over time in the same manner.

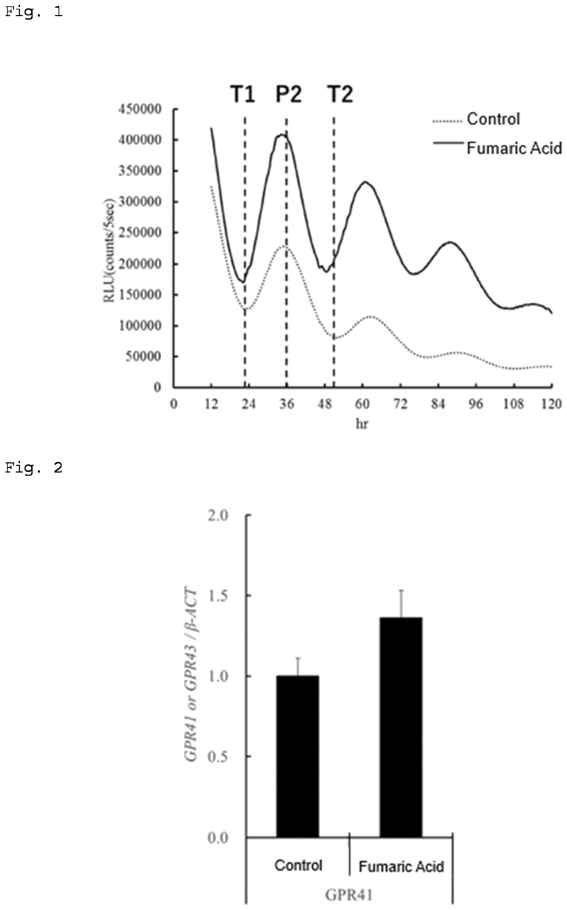

Fig. 1 shows the measurement results. In the luminescence intensity rhythms, valleys were represented by T and peaks by P, and they were labeled as P1, T1, P2, and T2 in order of appearance. Since P1 was unstable in rhythm, the circadian rhythm of per2 reporter cells was evaluated based on the phases of the valleys (T1 and T2) and peak (P2) after T1.

Table 1 summarizes the phase difference (in hours) between the circadian rhythm of per2 reporter cells with fumaric acid added (fumaric acid-added cells) and the circadian rhythm of control per2 reporter cells (control), based on the measurement results shown in Fig. 1.

**Table 1: Difference in Phase Between Fumaric Acid-added Cells and Control**

| | **T1** | **P2** | **T2** |
|---|---|---|---|
| **Control** | **22.4** | **34.8** | **52.0** |
| **Fumaric Acid-added Cells** | **22.2** | **34.2** | **48.6** |
| **Difference in Phase** | **-0.2** | **-0.6** | **-3.4** |
| | | | **(hr)** |

As shown in Fig. 1 and Table 1, a comparison with the control indicated that the addition of fumaric acid advances the phase of the circadian rhythm. From this fact, fumaric acid was confirmed to have the action of regulating the circadian rhythm by advancing the phase of the circadian rhythm.

Additionally, Table 2 shows the results of a comparison of circadian rhythm cycle (hours) between the fumaric acid-added cells and the control. The cycle was calculated from the phase difference between T1 and T2 (T2-T1).

**Table 2**

| **Table 2: Difference in Cycle Between Fumaric Acid-added Cells and Control** | |
|---|---|
| | **T2-T1** |
| **Control** | **29.6** |
| **Fumaric Acid-added Cells** | **26.4** |
| **Difference in Cycle** | **-3.2** |
| | **(hr)** |

As shown in Fig. 1 and Table 2, a comparison with the control indicated that the addition of fumaric acid shortens the cycle of the circadian rhythm. From this fact, fumaric acid was confirmed to have the action of regulating the circadian rhythm by shortening the cycle of the circadian rhythm.

### Experimental Example 2: Induction of GPR41 Expression by Fumaric Acid

Caco-2 cells were seeded in a 24-well plate at 2 × 10⁵ cells/well. After 3 weeks of differentiation induction, 5 mM fumaric acid was added, and cells were collected 60 minutes later. RNA was extracted, and GPR41 gene expression levels were measured by real-time PCR. Fig. 2 shows the results.

As shown in Fig. 2, the expression level of the GPR41 gene increased due to the addition of fumaric acid. This suggests that fumaric acid may have the action of promoting GLP-1 secretion through the expression of the GPR41 gene, thereby regulating the circadian rhythm of peripheral tissue by insulin.

### Industrial Applicability

The present disclosure provides highly safe food-derived ingredients or materials whose safety has been confirmed, as circadian regulatory agents, thereby preventing or ameliorating physical discomfort caused by disrupted circadian rhythms, as well as supporting health maintenance and improvement and enhancing work efficiency for people before the onset of disease.

## Claims

1. A composition for regulating a circadian rhythm, comprising as an active ingredient at least one selected from the group consisting of fumaric acid and salts thereof.

2. The composition for regulating a circadian rhythm according to claim 1, which is for use in advancing the phase of the circadian rhythm and/or in shortening the cycle of the circadian rhythm.

3. The composition for regulating a circadian rhythm according to claim 1 or 2, comprising a fermented material of a fumaric acid-producing bacterium or a processed product thereof, the fermented material or the processed product thereof containing at least one selected from the group consisting of fumaric acid and salts thereof.

4. The composition for regulating a circadian rhythm according to claim 3, wherein the fumaric acid-producing bacterium is at least one species of lactic acid bacteria selected from the group consisting of *Streptococcus thermophilus* and *Lactiplantibacillus plantarum.*

5. The composition for regulating a circadian rhythm according to claim 1 or 2, which is a composition for regulating a daily rhythm.

6. The composition for regulating a circadian rhythm according to claim 1 or 2, which is a food or drink, a quasi-drug, a drug, or an additive therefor.

7. The composition for regulating a circadian rhythm according to claim 3, which is a food or drink, a quasi-drug, a drug, or an additive therefor.

8. The composition for regulating a circadian rhythm according to claim 1 or 2, not comprising a composition containing either or both of the following compounds (1) and (2):
(1) a fumarate salt of (1R,2S) -2-(((2,4-dimethylpyrimidin-5-yl)oxy)methyl)-2-(3-fluorophenyl)-N-(5-fluoropyridin-2-yl)cyclopropanecarboxamide, and
(2) a fumarate salt of ornithine.
